(19) Europäisches Patentamt
European Patent Office
Office européen des brevets

(11) **EP 0 946 870 B1**

(12) # FASCICULE DE BREVET EUROPEEN

(45) Date de publication et mention de la délivrance du brevet:
**27.11.2002 Bulletin 2002/48**

(21) Numéro de dépôt: **97951325.6**

(22) Date de dépôt: **12.12.1997**

(51) Int Cl.[7]: **G01N 33/533**, G01N 33/58

(86) Numéro de dépôt international:
**PCT/FR97/02288**

(87) Numéro de publication internationale:
**WO 98/026287 (18.06.1998 Gazette 1998/24)**

(54) **CONJUGUES FLUORESCENTS NON AGREGES ET LEUR PROCEDE D'OBTENTION**

NICHT AGGREGIERTE FLUORESZIERENDE KONJUGATE UND VERFAHREN ZU IHRER HERSTELLUNG

NON-AGGREGATED FLUORESCENT CONJUGATES AND METHOD FOR OBTAINING THEM

(84) Etats contractants désignés:
**AT BE CH DE DK ES FI FR GB GR IE IT LI LU MC NL PT SE**

(30) Priorité: **12.12.1996 FR 9615261**

(43) Date de publication de la demande:
**06.10.1999 Bulletin 1999/40**

(73) Titulaire: **CIS BIO INTERNATIONAL**
**91400 Saclay (FR)**

(72) Inventeur: **ASPE, Daniel**
**F-30290 Laudun (FR)**

(74) Mandataire: **Gillard, Marie-Louise et al**
**Cabinet Beau de Loménie,**
**158, rue de l'Université**
**75007 Paris (FR)**

(56) Documents cités:
**WO-A-91/02040**
**WO-A-91/05605**
**WO-A-95/02700**
**US-A- 5 068 227**
**US-A- 5 268 486**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 946 870 B1

Printed by Jouve, 75001 PARIS (FR)

## Description

L'invention concerne un procédé de fabrication de conjugués fluorescents non agrégés. L'invention concerne également les conjugués fluorescents non agrégés obtenus par ce procédé et leur utilisation comme traceurs fluorescents.

L'autoagrégation de colorants hydrophobes tels que la thionine ou le bleu de méthylène, est bien connue en solution aqueuse à haute concentration, comme décrit dans J. Am. Chem. Soc. 63,69 (1941). Ces agrégats entraînent un changement de spectre d'absorption et une réduction de la fluorescence desdits colorants.

Les cyanines sont également connues pour s'agréger en provoquant un quenching de fluorescence (J. Phys. Chem. 69, 1894 (1965)).

Ce phénomène d'agrégation est notamment amplifié dans la fabrication de conjugués fluorescents. Waggoner et al. ont ainsi observé un phénomène d'agrégation après conjugaison de cyanine isothiocyanate sur un anticorps (Cytométrie 10:11-19 (1989)). Ces mêmes auteurs décrivent des cyanines arylsulfonates présentant la particularité de se coupler par voie N-hydroxysuccinimide et de s'agréger faiblement sur conjugués protéiques, propriété qu'ils attribuent à la présence de groupements sulfonates sur le noyau cyanine (brevet US 5 268 486), ainsi que des cyanines à base de naphtalènesulfonate (Bioconjugate Chemistry 7 : 356-362 (1996)). Dans ce dernier article, les auteurs montrent l'importance du nombre de sulfonates dans les processus de désagrégation.

Cependant, il a été rapporté que la fluorescence d'un conjugué entre une cyanine désignée par CY5.18 issue du brevet US 5 268 486 et un anticorps anti-HCG (rapport molaire = 1,7) est quenchée par rapport à celle de la cyanine libre (Anal. Biochem. 217: 197-204 (1994)).

D'autre part, la Demanderesse a réalisé des essais sur des anticorps anti-cancer prostatique et anti-hormone spécifique de la thyroïde marqués avec les composés CY5.18 et CY5.29 issus du même brevet US 5 268 486 (exemples 1 et 2). Ces exemples montrent un fort quenching des cyanines quelque soit le taux de marquage final .

Enfin, dans le même brevet US 5 268 486 , les auteurs soulignent l'importance des cyanines comme remplacant potentiel des phycobiliprotéines et mettent en avant les avantages suivants :

- stabilité
- coût moindre
- procédure de marquage simplifiée
- taille adaptée à la reconnaissance de petites molécules.

Ces avantages deviennent toutefois inopérants quand les cyanines se retrouvent agrégées sur les conjugués.

La demande WO 96/00902 propose, pour pallier l'inconvénient de l'agrégation des cyanines, d'introduire des groupements iminium dans leur structure. Toutefois, cette demande se contente d'apprécier la capacité désagrégeante de structures qui y sont décrites en comparant les spectres UV dans des solutions salines faiblement et fortement concentrées, et ne donne aucun résultat sur les conjugués.

Parallèlement, l'augmentation de fluorescence provoquée par l'ajout de cylodextrine dans un milieu contenant un fluorophore est décrite dans la littérature (J. Chromatog. 452 (1988); Macromolecules 10(3) : 676-681 (mai-juin 1977)).

Inversement, certaines molécules fluorescentes sont quenchées par l'addition de cyclodextrines (Journal of Inclusion Phenomena and Molecular Recognition in Chemistry 18 : 385-396 (1994), Kluwer Academic Publishes).

La formation de complexes d'inclusion cyanine-cyclodextrine est décrite en littérature (J. Am. Chem. Soc. 112 : 5824-5830 (1990)). Selon les conditions et la nature de la cyclodextrine, on peut voir des complexes monomériques ou dimériques, lesquels favorisent l'agrégation des cyanines et donc le quenching de fluorescence desdites cyanines.

Pour conserver les propriétés fluorescentes des complexes d'inclusion, l'art antérieur décrit des liaisons covalentes faisant intervenir une fonction de la cyclodextrine (WO 91/02040).

La demande WO 91/01090 décrit des chélates d'Europium attachés de façon covalente à une cyclodextrine, qui est ensuite couplée à une protéine.

Toutefois, ce type de liaison est difficile à réaliser, notamment parce qu'elle nécessite la modification chimique de la cyclodextrine (sous forme par exemple de N-hydroxysuccinimide, maléimide, thiol ou amine) et présente l'inconvénient :

- d'altérer les propriétés des complexes d'inclusion et donc de diminuer les performances de fluorescence ;
- d'altérer les propriétés de la protéine, par un marquage de la cyclodextrine sur des fonctions biodisponibles.

La présente invention se propose de remédier à ces inconvénients et de préserver les propriétés de conjugués entre une molécule porteuse et un réactif fluorophore.

Ainsi, un premier objet de l'invention consiste à obtenir un conjugué hautement fluorescent et non agrégé.
Un autre objet de l'invention consiste à obtenir un tel conjugué fluorescent de manière simple et rapide.
Ces objets sont atteints, selon un premier aspect de l'invention, par un procédé pour l'obtention d'un conjugué fluorescent entre une molécule porteuse possédant au moins un groupement amino, hydroxy, carboxy et/ou sulfhydryl et un réactif fluorophore possédant au moins un groupement fonctionnel susceptible de réagir avec le(s)dit(s) groupe(s) amino, hydroxy, carboxy et/ou sulfhydryl, qui consiste à mettre en présence ladite molécule porteuse et ledit réactif fluorophore avec une solution aqueuse d'un macrocycle soluble dans l'eau contenant de 1 à 40 % (m/v) dudit macrocycle.
Le mécanisme réactionnel peut être représenté par le schéma suivant :

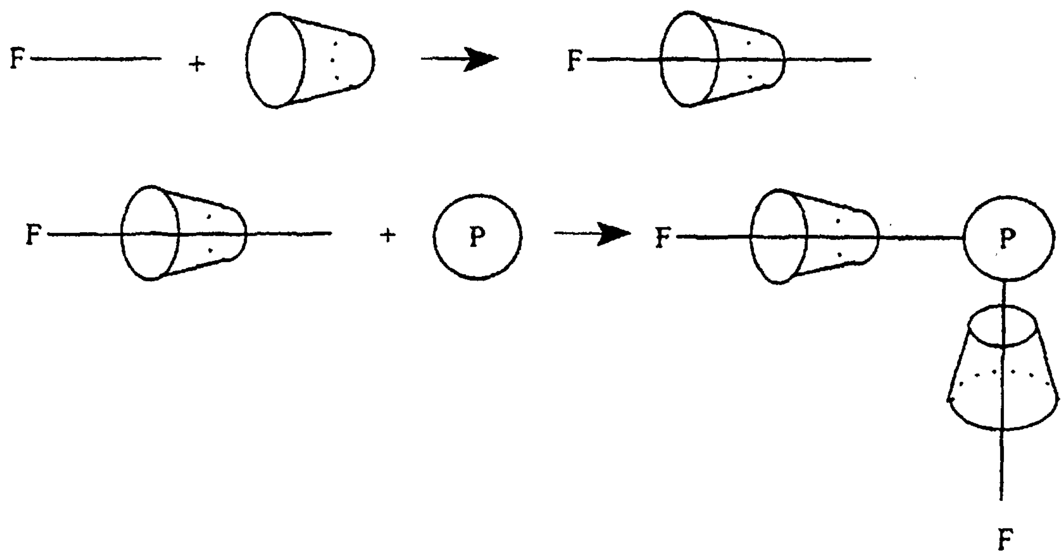


où

F est le réactif fluorophore et
P est la molécule porteuse.

La molécule porteuse P réagit avec les fonctions du réactif fluorophore F. Cela a pour effet de conserver les distances intermoléculaires et d'éviter le rapprochement des réactifs fluorophores sur la molécule porteuse. La conséquence en est une diminution de l'agrégation des réactifs fluorophores.
Avantageusement, la concentration en macrocycle dans la solution aqueuse de macrocycle est comprise entre 10 et 40 % (m/v).
Comme macrocycle soluble dans l'eau, on utilisera avantageusement une cyclodextrine éventuellement substituée ou un calixarène substitué par des groupes hydrophiles.
On peut citer à titre d'exemple de cyclodextrine utilisable dans le procédé conforme à l'invention, l'α-cyclodextrine, la β-cyclodextrine, la γ-cyclodextrine, l'hydroxypropyl-α-cyclodextrine, l'hydroxypropyl-β-cyclodextrine, l'hydroxypropyl-γ-cyclodextrine, l'hydroxyéthyl-α-cyclodextrine, l'hydroxyéthyl-β-cyclodextrine, l'hydroxyéthyl-γ-cyclodextrine ou la 2,6-di-O-méthyl-heptakis-β-cyclodextrine.
Les calixarènes qui peuvent être utilisés dans le procédé conforme à l'invention répondent à la structure

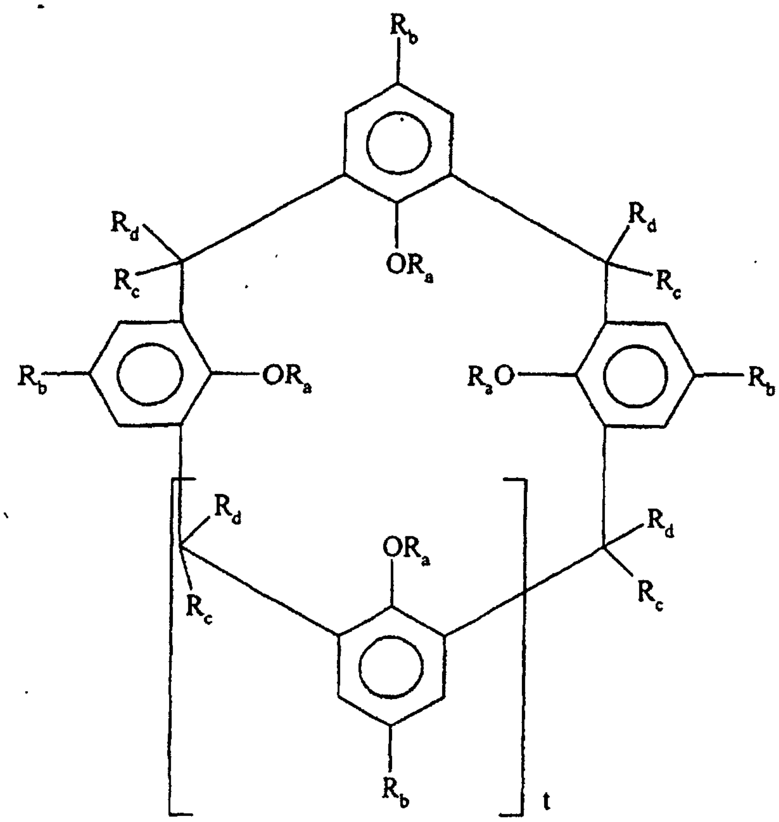

dans laquelle :

- $R_a$ et $R_b$ représentent chacun un groupement hydrophile choisi parmi H, $(CH_2)_pCO_2H$, $(CH_2)_pOH$, $(CH_2)_pNH_2$, $(CH_2)_pSO_3H$ ou $(CH_2)_pN^+R_cR_dR_e$;
- $R_c$, $R_d$ et $R_e$ représentent chacun l'hydrogène ou un $(C_1-C_3)$alkyle ;
- p varie de 0 à 4 pour $R_b$ et de 1 à 4 pour $R_a$ ; et
- t varie de 1 à 5.

Le macrocycle soluble dans l'eau forme un complexe d'inclusion, soit avec le réactif fluorophore, soit avec la molécule porteuse et forme ainsi une structure rotaxane stable. Ce complexe d'inclusion peut subsister dans la structure finale du conjugué fluorescent obtenu par le procédé conforme à l'invention.

Généralement, le réactif fluorophore utilisé est un chromophore à un ou plusieurs noyaux aromatiques, ledit chromophore ayant un coefficient d'extinction moléculaire élevé, supérieur à 20 000, de préférence supérieur à 50 000.

Selon un aspect préféré de l'invention, ledit réactif fluorophore est choisi parmi

* une cyanine de structure

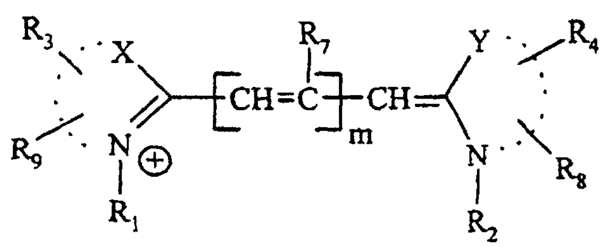

;

* une hémicyanine de structure

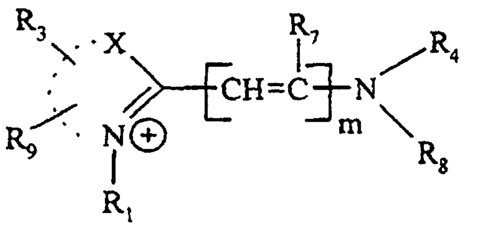

;

* une mérocyanine de structure

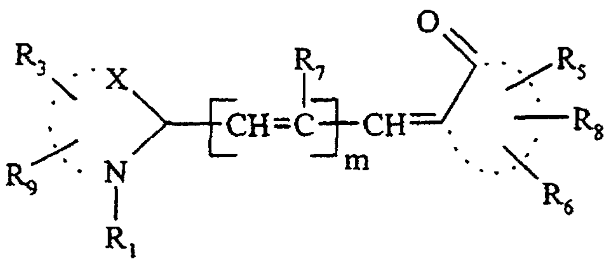

ou

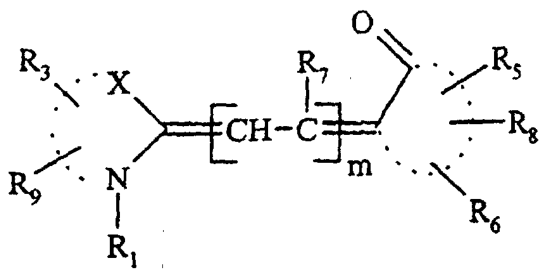

;

* un styryl de structure

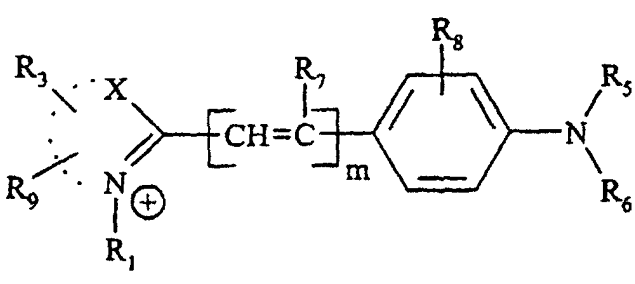

;

* une rhodamine de structure :

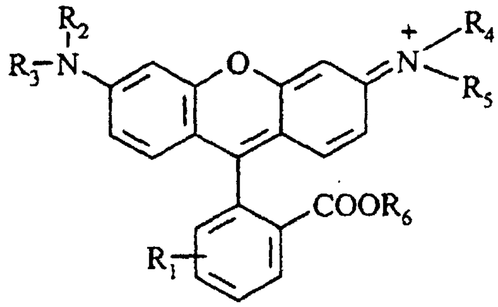

ou

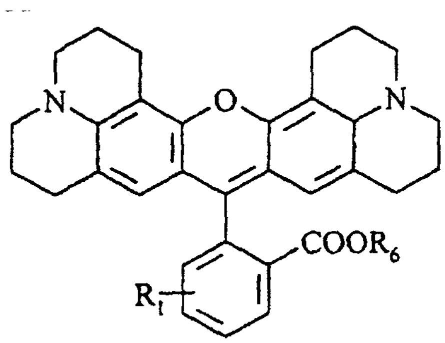

;

* une fluorescéine de structure :

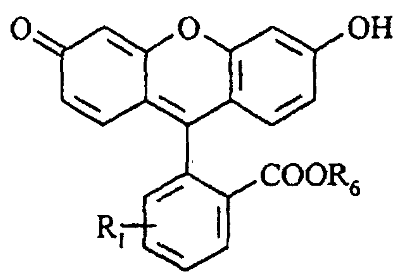

;

ou

* une naphtofluorescéine de structure :

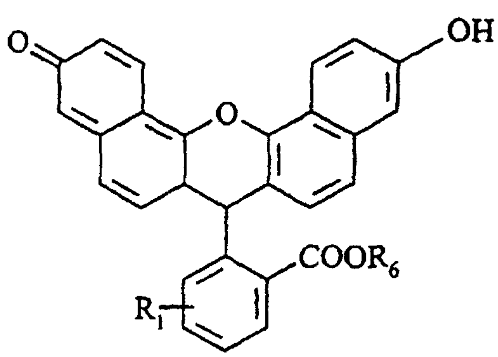

;

dans lesquelles structures :

- les lignes en pointillé représentent chacune les atomes de carbone nécessaires pour former 1 à 3 cycles fusionnés, les groupes $R_3$, $R_4$, $R_5$, $R_6$, $R_8$ et $R_9$ étant attachés à ces cycles ;
- X et Y représentent chacun N,

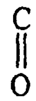 ,

O, S ou $C(CH_3)_2$ ;
- m vaut 1, 2, 3 ou 4;
- au moins un des groupes $R_1$ à $R_7$ est capable de réagir avec un groupement amino, hydroxy, carboxy et/ou sulfhydryl et est choisi parmi

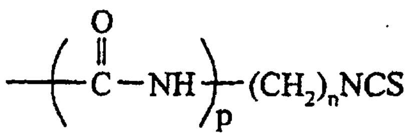 ; 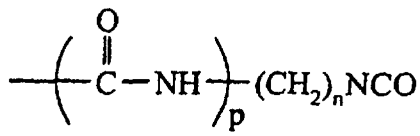 ;

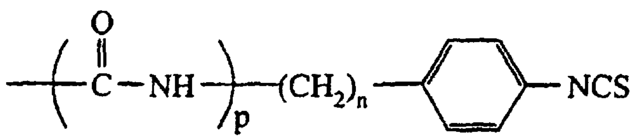 ;

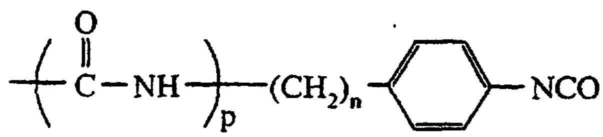 ;

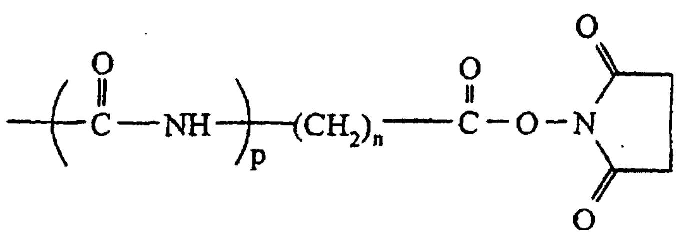 ;

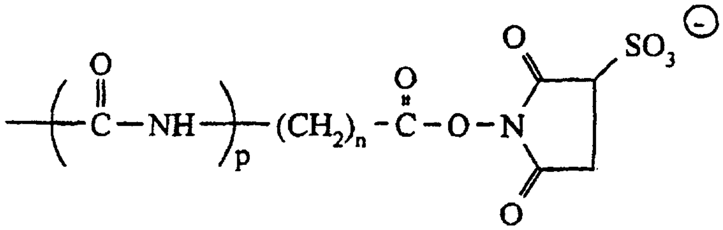 ;

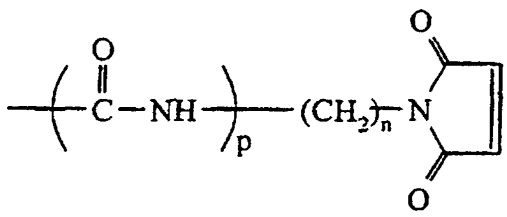 ;

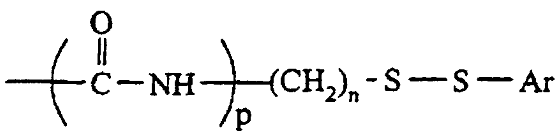 ; 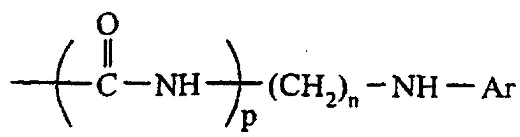 ;

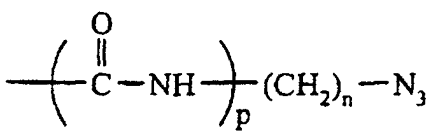 ; 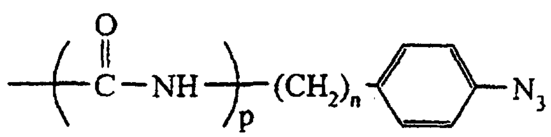 ;

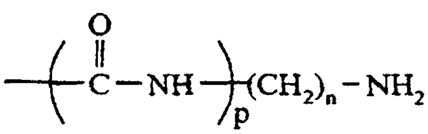 ou 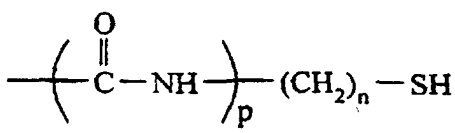

où n varie de 0 à 8 et p est égal à 0 ou 1, et Ar est un hétérocycle à 5 ou 6 chaînons comprenant 1 à 3 hétéroatomes, éventuellement substitué par un atome d'halogène, ceux des groupes $R_1$ à $R_7$ ne représentant pas une des entités réactives ci-dessus étant choisis parmi l'hydrogène ou un groupe $-(CH_2)_r-Z$ dans lequel r varie de 0 à 4 et Z représente un groupe $CH_3$, $SO_3H$, OH ou $N^+R_1R_2R_3$ dans lequel $R_1$, $R_2$ et $R_3$ sont tels que définis ci-dessus, et au moins un des groupes $R_8$ et $R_9$ représente un groupe $SO_3^-$ ou $SO_3H$, l'autre groupe représentant l'hydrogène ou un groupe $SO_3^-$ ou $SO_3H$. De préférence, p est égal à 0 dans les formules ci-dessus.

Plus particulièrement, on utilisera de manière avantageuse une cyanine de structure

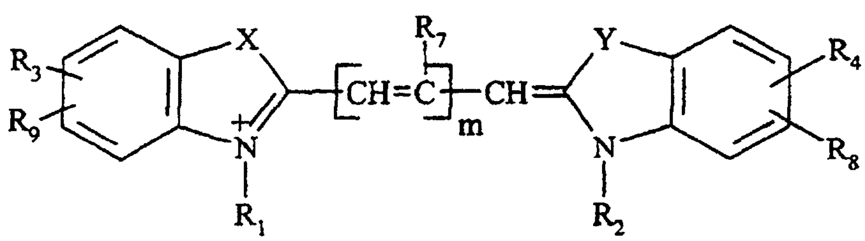

dans laquelle les groupes $R_1$, $R_2$, $R_3$, $R_4$, $R_7$, $R_8$ et $R_9$ sont tels que définis précédemment.

Une classe de cyanines préférée est composé des cyanines ci-dessus dans lesquelles X et Y représentent chacun un groupe $C(CH_3)_2$.

Parmi ces cyanines, celles où

(i) $R_1$ et $R_2$ représentent chacun un groupe 5-(succinimidooxycarbonyl)pentyl ; $R_3$, $R_4$ et $R_7$ représentent chaucn l'hydrogène ; $R_8$ et $R_9$ représentent chacun un groupe sulfonate ; et m est égal à 2 ou
(ii) $R_1$ représente un groupe 5-(succinimidooxycarbonyl)pentyl; $R_2$ représente un éthyl ; $R_3$, $R_4$ et $R_7$ représentent chaucn l'hydrogène ; $R_8$ et $R_9$ représentent chacun un groupe sulfonate ; et m est égal à 2, sont préférés.

La molécule porteuse possédant au moins un groupement amino, hydroxy, carboxy et/ou sulfhydryl est une

biomolécule destinée d'une manière générale au diagnostic ou à la détection. Cette biomolécule pourra servir elle-même au marquage d'autres molécules, notamment de protéines. A titre d'exemple, on peut citer un anticorps, un antigène, une protéine, un peptide, un haptène, une lectine, l'avidine, la streptavidine, une toxine, un glucide, un oligosaccharide, un polysaccharide, un acide nucléique, une hormone, un médicament, un polymère, une particule polymérique, du verre, une particule de verre ou une surface en verre ou en polymère.

Le procédé conforme à l'invention présente les avantages suivants :

- il évite la fonctionnalisation de cyclodextrines, et permet donc un gain de temps et d'argent ;
- il permet la stabilisation de façon irréversible du complexe d'inclusion entre le macrocycle soluble dans l'eau et le réactif fluorophore (les complexes d'inclusion ont habituellement des constantes d'équilibre faibles) ;
- il permet d'éviter le quenching de fluorescence des conjugués obtenus, sans intervenir sur la structure chimique du réactif fluorophore. En effet, les propriétés complexantes du macrocycle soluble dans l'eau permettent un éloignement *in situ* des réactifs fluorophores dont les distances intermoléculaires après conjugaison et purification du conjugué sont maintenues. Cet éloignement des réactifs fluorophores sur la molécule porteuse évite le phénomène d'agrégation et donc le quenching de fluorescence.

Selon un autre aspect, l'invention concerne également les conjugués fluorescents, obtenus par le procédé décrit précédemment.

Lorsque le complexe d'inclusion formé par le macrocycle soluble dans l'eau avec le réactif fluorophore et la molécule porteuse subsiste dans la structure finale du conjugué fluorescent, le réactif fluorophore est lié à la molécule porteuse de telle sorte que la partie hydrophobe du réactif fluorophore traverse le macrocycle soluble dans l'eau.

Si la molécule porteuse P et le réactif fluorophore F sont des groupements encombrants, le complexe réalisé sera stable et formera un rotaxane:

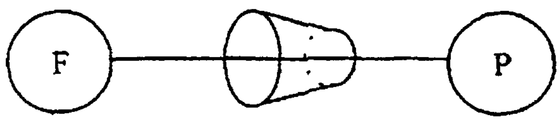


Le complexe obtenu est original car la cyclodextrine ne contient aucune liaison covalente avec le réactif fluorophore ou la molécule porteuse.

Comme indiqué précédemment, les conjugués fluorescents selon l'invention, notamment les complexes rotaxane, ne présentent pas de quenching de fluorescence en raison de l'éloignement des réactifs fluorophores sur la molécule porteuse.

Les conjugués fluorescents selon l'invention se révèlent être d'excellents traceurs fluorescents.

Ainsi, selon un autre aspect, l'invention concerne l'utilisation des conjugués fluorescents décrits ci-dessus comme traceurs fluorescents. Ces traceurs trouvent application par exemple en microscopie de fluorescence, en cytométrie de flux ou en immunodiagnostic en fluorescence, de préférence en microscopie de fluorescence ou en cytométrie de flux. Les conjugués fluorescents de l'invention peuvent également être utilisés pour la détection et/ou la détermination par fluorescence d'un analyte dans un milieu susceptible de le contenir.

L'invention sera mieux comprise à l'aide des exemples ci-après, donnés à titre purement illustratifs.

EXEMPLE 1 - Fabrication des conjugués et analyse UV

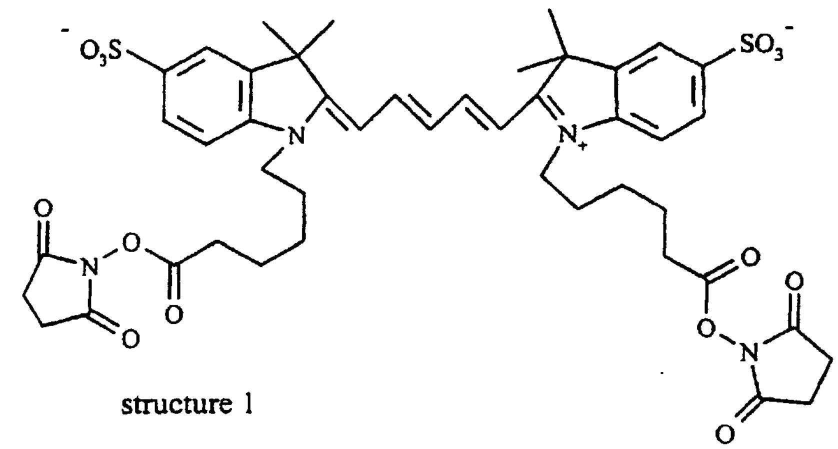

structure 1

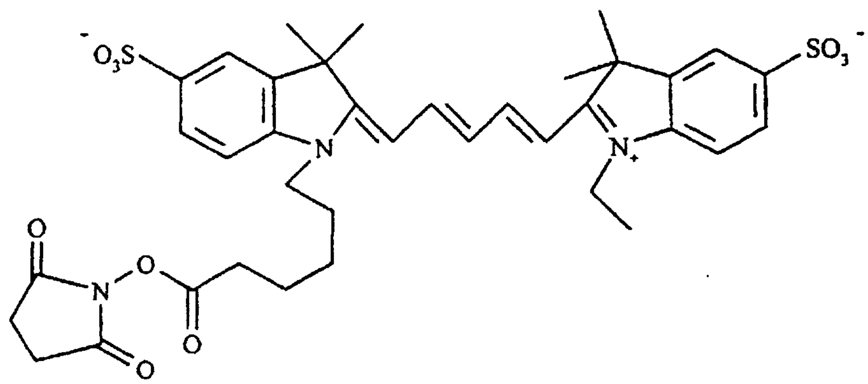

structure 2

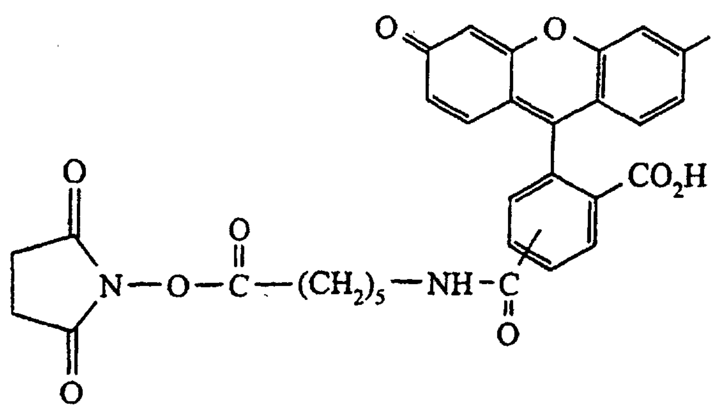

structure 3

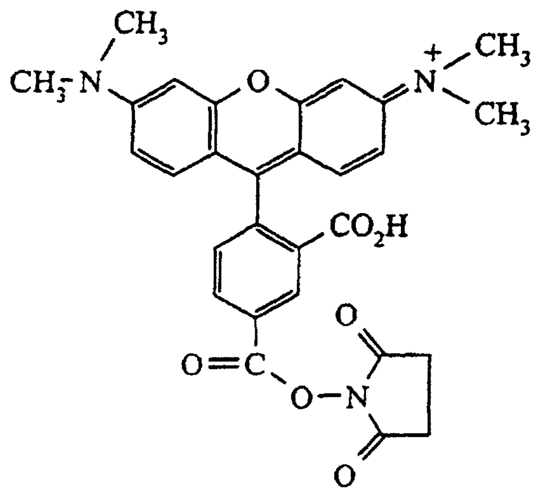

structure 4

1/ influence de la présence de la cyclodextrine

*Conjugué N° 1*

Un échantillon à 1 mg/ml d'anticorps marqueur tumoral du pancréas (référence : CA 19-9) en tampon carbonate 0,1M pH 9 est mélangé à température ambiante avec un excès molaire de 17 pour 1 de sulfoindodicarbocyanine de structure 1. Au bout de deux heures, l'échantillon est purifié par filtration sur gel Séphadex ®G25.
Le rapport molaire cyanine/anticorps final est de 3,90.
On observe des raies en UV à 650 et 605 nm. Le rapport $DO_{650}/DO_{605}$ est de 1,62. L'apparition de la raie à 605 nm est signe d'une agrégation et d'un quenching de la molécule fluorescente.

*Conjugué N° 2*

On prépare une solution tampon carbonate 0,1 M pH 9 dans laquelle on dissout 40 % (m/v) d'hydroxypropyl-β-cyclodextrine.
L'anticorps utilisé dans la préparation du conjugué n° 1 est dialysé dans ce tampon, auquel on ajoute une solution de sulfoindodicarbocyanine de structure 1.
Le rapport molaire initial cyanine/anticorps est de 17 pour 1.
Au bout de deux heures, l'échantillon est purifié sur gel Séphadex ® G25.
On analyse par UV. On observe des raies à 650 et 605 nm.
On obtient un rapport molaire final cyanine/anticorps de 5,89 et un rapport $DO_{650}/DO_{605}$ de 3,18.
Cet exemple montre bien que l'utilisation du complexe cyanine-cyclodextrine a permis d'éviter le phénomène d'agrégation constaté pour le conjugué N° 1.

*Conjugué N° 3*

Un anticorps anti-PSA (antigène spécifique de la prostate, de l'anglais "Prostate Specific Antigen", référence : PSA) est dialysé dans du tampon carbonate 0,1 M pH 9.
La solution à 0,85 mg/ml en anticorps est mise en contact avec la sulfoindodicarbocyanine de structure 1. Le rapport molaire initial cyanine/anticorps est de 15 pour 1.
Deux heures après, on purifie sur gel Séphadex ® G25.
Le produit est analysé par UV. On observe des raies à 650 et 605 nm. On obtient un rapport molaire final cyanine/anticorps de 4,54 et un rapport $DO_{650}/DO_{605}$ de 1.40.

*Conjugué N° 4*

On procède comme décrit pour l'obtention du conjugué n° 3, excepté qu'on rajoute 15 % (m/v) d'hydroxypropyl-β-cyclodextrine dans le tampon carbonate 0,1 M pH 9.
Le conjugué analysé en UV donne les résultats suivants :

$$DO_{650}/DO_{605} = 2,60$$

Rapport molaire final cyanine/anticorps = 0,96

En comparant au conjugué N° 3 on note une forte augmentation du rapport $DO_{650}/DO_{605}$. Cela traduit une réduction importante de l'agrégation et un quenching sensiblement diminué.

*Conjugués N° 5 et 6*

On opère respectivement comme pour les conjugués N° 1 et 2, en utilisant un anticorps anti-hormone chorionique gonadotrope à une concentration de 3 mg/ml et, comme marqueur fluorescent, la fluorescéine de structure 3. Le pH de couplage est 9 et le temps d'incubation de 30 mn.

On observe en UV des raies à 497 et 462 nm.

Les résultats sont rassemblés dans le Tableau 1 :

Tableau 1

| N° conjugué | rapport molaire initial fluorescéine/anticorps | rapport molaire final fluorescéine/anticorps | % cyclodextrine | $DO_{497}/DO_{462}$ |
|---|---|---|---|---|
| 5 | 4 | 2,1 | 0 | 2 |
| 6 | 10 | 2,4 | 40 | 2,3 |

La présence de cyclodextrine, pendant le couplage du dérivé fluorescéine, a permis d'augmenter le rapport des densités optiques $DO_{497}/DO_{462}$, et donc de diminuer le phénomène d'agrégation. (Le spectre UV du dérivé fluorescéine seul présente un rapport de raies égal à 2,4).

Le conjugué N° 6 est plus performant en terme de fluorescence.

2/ Influence de la nature de la cyclodextrine

Différentes cyclodextrines (conjugués 7 à 12) sont testées afin d'apprécier l'influence de la nature de la cyclodextrine sur l'apparition des raies parasites : 605 nm pour la cyanine et 522 nm pour la rhodamine. Deux conjugués témoins (conjugués N°13 et 14) ne contenant pas de cyclodextrine sont aussi testés à titre de comparaison.

Les conjugués 7 à 10 et 13 sont obtenus en suivant le mode opératoire décrit pour l'obtention du conjugué n° 2, en appliquant les conditions expérimentales suivantes :

nature de l'anticorps : anticorps anti-PSA
concentration de l'anticorps : 0,75 mg/ml
concentration de la cyclodextrine dans le tampon carbonate 0,1 M pH 9 : 13 (ou 0) % (m/v)
rapport molaire initial cyanine/anticorps : 17 pour 1
temps d'incubation : 2 h

Après deux heures, la cyanine libre est épuisée sur cône amicon par centrifugation pendant 5 min. à 4000 g. On purifie ensuite sur gel Séphadex ® G25.

Les conjugués 11, 12 et 14 sont obtenus en suivant le mode opératoire décrit pour l'obtention du conjugué N° 2, en appliquant les conditions expérimentales suivantes :

marqueur fluorescent : rhodamine de structure 4
nature de l'anticorps : anticorps anti-hormone chorionique gonadotrope
concentration de l'anticoprs : 4 mg/ml
concentration de la cyclodextrine dans le tampon carbonate 0,1 M pH 9: 10 (ou 0) % (m/v)
rapport molaire initial rhodamine/anticorps : 17 pour 1
temps d'incubation : 30 mn.

Les résultats sont rassemblés dans le Tableau 2 :

Tableau 2

| | conjugué N°7 hydroxypropyl-α-cyclodextrine | conjugué N°8 hydroxypropyl-γ-cyclodextrine | conjugué N°9 hydroxypropyl-β-cyclodextrine | conjugué N°10 diméthyl-β-cyclodextrine | conjugué N° 11 hydroxypropyl-β-cyclodextrine | conjugué N° 12 diméthyl-β-cyclodextrine | conjugué N° 13 - | conjugué N° 14 - |
|---|---|---|---|---|---|---|---|---|
| rapport molaire final cyanine (rhodamine)/ anticorps | 1,8 | 1,8 | 1,2 | 2,0 | 1 | 1,2 | 2,6 | 1,3 |
| $DO_{650}/DO_{605}$ * $DO_{533}/DO_{522}$ | 1,9 | 2,0 | 2,6 | 2,55 | 2,4* | 1,54* | 1,45 | 1,37* |
| Fluorophore | cyanine | cyanine | cyanine | cyanine | rhodamine | rhodamine | cyanine | rhodamine |

Les fortes valeurs du rapport $DO_{650}/DO_{605}$ (2,6 et 2,55) obtenues avec les deux β-cyclodextrines montrent leur capacité à désagréger la cyanine de structure 1. Ces valeurs sont aussi le reflet de constantes de stabilité élevées.
Pour le dérivé rhodamine de structure 4, l'utilisation d'hydroxypropyl-β-cyclodextrine permet d'augmenter de façon sensible le rapport des densités optiques et de diminuer en conséquence l'effet d'agrégation.

3/ Influence de la concentration de couplage

Les concentrations sont un élément important dans les phénomènes d'agrégation.
Différentes concentrations en anticorps sont donc testées.
Les conjugués sont obtenus en suivant le mode opératoire décrit pour l'obtention du conjugué n° 2, en appliquant les conditions expérimentales suivantes :

nature de l'anticorps : anticorps asti-PSA
rapport molaire initial cyanine/anticorps : 17 pour 1
temps de couplage : 2 h
nature de la cyclodextrine : diméthyl-β-cyclodextrine
concentration en cyclodextrine dans le tampon carbonate 0,1 M pH 9 : 13 %(m/v)

Les résultats sont rassemblés dans le Tableau 3.

Tableau 3

| | Conjugué N°15 | Conjugué N°16 | Conjugué N°17 |
|---|---|---|---|
| concentration en anticorps (mg/ml) | 0,75 | 1,5 | 3,0 |
| rapport molaire final cyanine/anticorps | 1,2 | 1,8 | 2,3 |
| $DO_{650}/DO_{605}$ | 2,7 | 2,6 | 2,45 |

Avec une concentration de 3 mg/ml on obtient un rapport molaire final cyanine/anticorps élevé (2,3) avec un bon rapport $DO_{650}/DO_{605}$ (2,45).
Ces valeurs sont à comparer à celles du Tableau 2 dans lequel un rapport molaire final cyanine/anticorps de 2,6 est associé à un rapport $DO_{650}/DO_{605}$ faible (1,45).
Le procédé selon l'invention permet donc d'augmenter le rapport molaire final cyanine/anticorps tout en conservant un bon rapport $DO_{650}/DO_{605}$.
Cela n'est pas possible sans l'utilisation de cyclodextrines.

4/ Influence de la concentration en cyclodextrine

Les conjugués sont obtenus en suivant le mode opératoire décrit pour l'obtention du conjugué n° 2, en appliquant les conditions expérimentales suivantes :

nature de l'anticorps : anticorps anti-TSH (hormone thyréotrope, de l'anglais "Thyroid-Stimulating Hormone", référence : TSH)
rapport molaire initial cyanine (de structure 2)/anticorps : 17 pour 1
temps de couplage : 1 h
nature de la cyclodextrine : hydroxypropyl-β-cyclodextrine

Après épuisement sur cône amicon, le conjugué est purifié sur gel Séphadex ® G25.
Les résultats sont rassemblés dans le Tableau 4.

Tableau 4

| | Conjugué N°18 | Conjugué N°19 | Conjugué N°20 |
|---|---|---|---|
| concentration en cyclodextrine (m/v) | 5 % | 14 % | 32 % |
| rapport molaire final cyanine/anticorps | 3,9 | 2,74 | 2,45 |
| $DO_{650}/DO_{605}$ | 1,7 | 2,2 | 2,6 |

On voit nettement dans le Tableau 4, l'influence du pourcentage de cyclodextrine.

La loi d'action de masse est déplacée dans le sens de formation du complexe par augmentation de la concentration de cyclodextrine.

EXEMPLE 2 : Performances de fluorescence des coniugués

L'analyte choisi est le PSA.

Les mesures sont effectuées sur un fluorimètre LS50 de PERKIN-ELMER (λmax d'émission = 660 nm).

Deux milieux sont testées :

- milieu 1 : tampon phosphate 0,1 M pH 7
- milieu 2 : milieu 1 + 1/3 sérum veau nouveau né

Le conjugué N° 21 a été fabriqué selon le mode opératoire décrit pour l'obtention du conjugué n° 2.

Les conjugués N° 22 et 23 ont été fabriqués selon le mode opératoire décrit pour l'obtention du conjugué n° 1 mais avec des concentrations d'anticorps respectivement de 0,1 et 0,3 mg/ml.

Les résultats sont rassemblés dans le Tableau 5.

TABLEAU 5

| | unités de fluorescence (a) | | | | rapport molaire final cyanine/anticorps | $DO_{650}/DO_{605}$ |
|---|---|---|---|---|---|---|
| | milieu 1 | | milieu 2 | | | |
| | excitation 600 nm | excitation 650 nm | excitation 600nm | excitation 650 nm | | |
| Conjugué n° 21 | 1 | 1 | 0,97 | 1,1 | 0,85 | 2,94 |
| Conjugué n° 22 | 0,12 | 0,26 | 0,13 | 0,28 | 1,0 | 2,5 |
| Conjugué n° 23 | 0,4 | 0,69 | 0,44 | 0,74 | 3,0 | 2,0 |

(a) les unités de fluorescence sont exprimées :
- par unité de densité optique
- en relatif par rapport au conjugué N° 21 dans le milieu 1.

L'examen du tableau 5 montre tout l'intérêt du procédé conforme à l'invention :

- dans tous les cas le conjugué fabriqué avec le complexe cyanine + cyclodextrine (conjugué N° 21) est supérieur en fluorescence aux conjugués fabriqués avec la cyanine sans cyclodextrine (conjugués N° 22 et 23).

- il n'y a pas en présence de sérum de quenching des conjugués fabriqués.

EXEMPLE 3 : Performance dans un immunoessai

L'immunoessai est conduit selon la méthode homogène décrite par G. Mathis dans Clin. Chem., Vol 39, n°9, 1993.
Les performances des conjugués 21, 22 et 23 ont comparées entre elles, en utilisant des échantillons standards à différentes concentrations d'antigène.
Les résultats sont rassemblés dans le Tableau 6 :

Tableau 6

| | conjugué n° 21 | conjugué n° 22 | conjugué n° 23 |
|---|---|---|---|
| échantillon N° 1 0,53 ng/ml | 113 | 67 | 86 |
| échantillon N° 2 5,3 ng/ml | 2380 | 1378 | 1885 |
| échantillon N° 3 31 ng/ml | 4175 | 2289 | 3323 |

Les grandeurs sont exprimées en unités de fluorescence
Ce tableau montre bien que le gain de fluorescence observé sur le conjugué 21 par rapport aux conjugués 22 et 23 (Tableau 5) se répercute également sur l'immunoessai.
Les conjugués fabriqués avec une cyclodextrine permettent donc d'augmenter la sensibilité des dosages dans un immunoessai.

**Revendications**

1. Procédé d'obtention d'un conjugué fluorescent entre une molécule porteuse possédant au moins un groupement amino, hydroxy, carboxy et/ou sulfhydryl et un réactif fluorophore possédant au moins un groupement fonctionnel susceptible de réagir avec le(s)dit(s) groupe(s) amino, hydroxy, carboxy et/ou sulfhydryl, qui consiste à mettre en présence ladite molécule porteuse et ledit réactif fluorophore avec une solution aqueuse d'un macrocycle soluble dans l'eau contenant de 1 à 40 % (m/v) dudit macrocycle.

2. Procédé selon la revendication 1, dans lequel le macrocycle soluble dans l'eau est choisi parmi une cyclodextrine éventuellement substituée ou un calixarène substitué par des groupements hydrophiles.

3. Procédé selon la revendication 2, dans lequel le macrocycle soluble dans l'eau est une cyclodextrine choisie parmi l'α-cyclodextrine, la β-cyclodextrine, la γ-cyclodextrine, l'hydroxypropyl-α-cyclodextrine, l'hydroxypropyl-β-cyclodextrine, l'hydroxypropyl-γ-cyclodextrine, l'hydroxyéthyl-α-cyclodextrine, l'hydroxyéthyl-β-cyclodextrine, l'hydroxyéthyl-γ-cyclodextrine, ou la 2,6-di-O-méthyl-heptakis-β-cyclodextrine.

4. Procédé selon la revendication 2, dans lequel le macrocycle soluble dans l'eau est un calixarène de structure

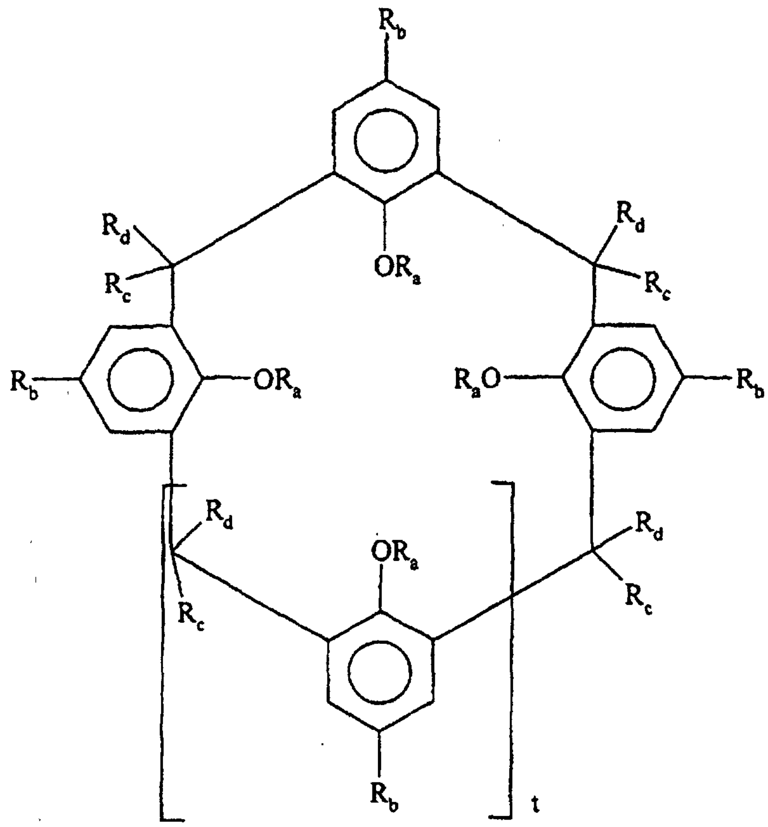

dans laquelle :

- $R_a$ et $R_b$ représentent chacun un groupement hydrophile choisi parmi H, $(CH_2)_pCO_2H$, $(CH_2)_pOH$, $(CH_2)_pNH_2$, $(CH_2)_pSO_3H$ ou $(CH_2)_pN^+R_cR_dR_e$ ;
- $R_c$, $R_d$ et $R_e$ représentent chacun l'hydrogène ou un $(C_1$-$C_3)$alkyle ;
- p varie de 0 à 4 pour $R_b$ et de 1 à 4 pour $R_a$ ; et
- t varie de 1 à 5.

**5.** Procédé selon l'une quelconque des revendications 1 à 4, dans lequel la solution aqueuse de macrocycle contient de 10 à 40 % (m/v) de macrocycle.

**6.** Procédé selon l'une quelconque des revendications 1 à 5, dans lequel le réactif fluorophore est un chromophore à un ou plusieurs noyaux aromatiques possédant un coefficient d'extinction moléculaire élevé.

**7.** Procédé selon la revendication 6, dans lequel le réactif fluorophore est choisi parmi

* une cyanine de structure

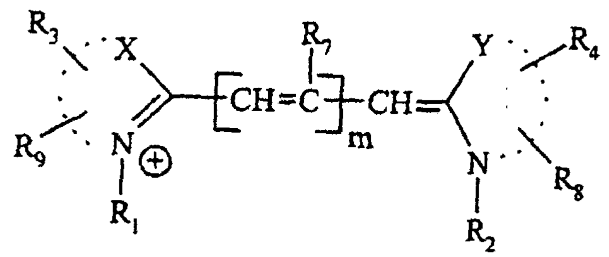

;

* une hémicyanine de structure

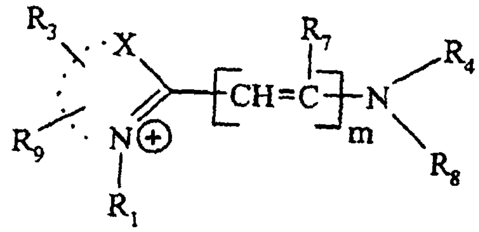

;

* une mérocyanine de structure

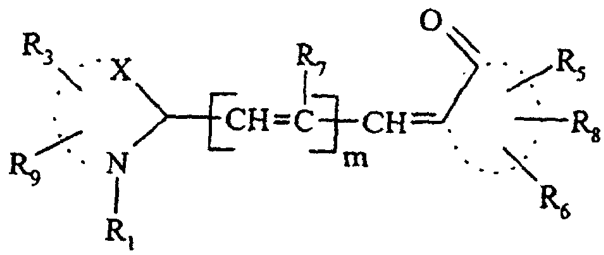

ou

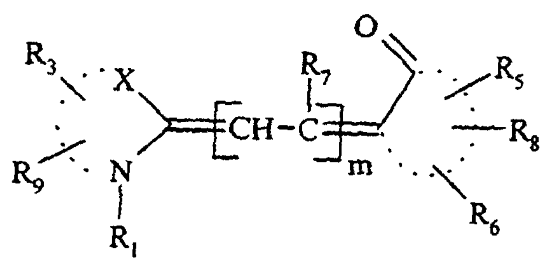

;

* un styryl de structure

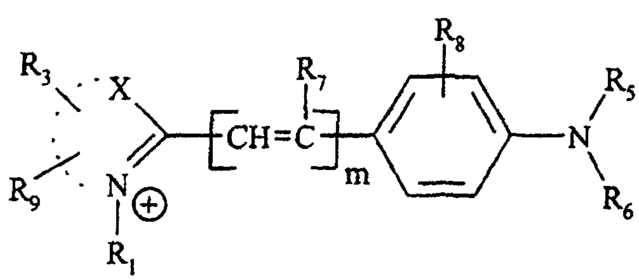

;

* une rhodamine de structure :

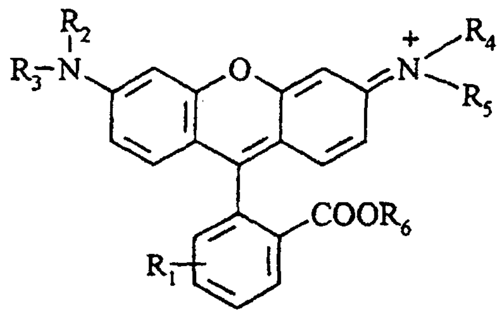

ou

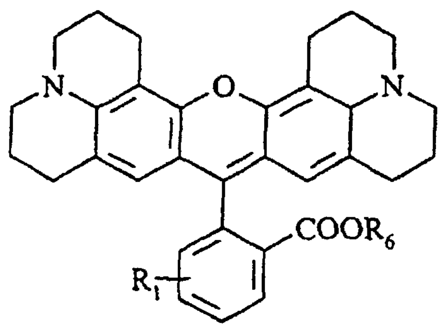

;

* une fluorescéine de structure :

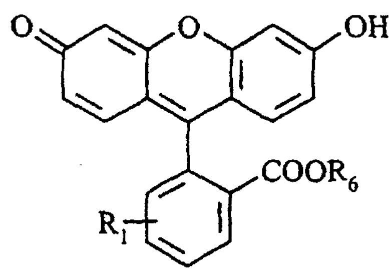

;

ou

* une naphtofluorescéine de structure :

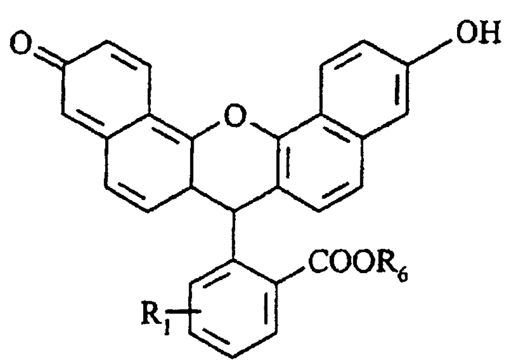

;

dans lesquelles structures :

- les lignes en pointillé représentent chacune les atomes de carbone nécessaires pour former 1 à 3 cycles fusionnés, les groupes $R_3$, $R_4$, $R_5$, $R_6$, $R_8$ et $R_9$ étant attachés à ces cycles ;
- X et Y représentent chacun N,

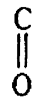,

  O, S ou $C(CH_3)_2$ ;
- m vaut 1, 2, 3 ou 4 ;
- au moins un des groupes $R_1$ à $R_7$ est capable de réagir avec un groupement amino, hydroxy, carboxy et/ou sulfhydryl et est choisi parmi

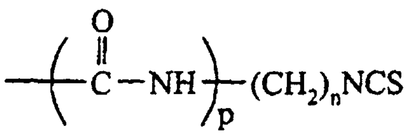 ; 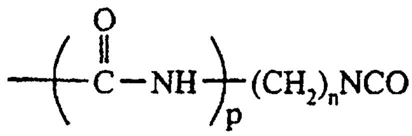 ;

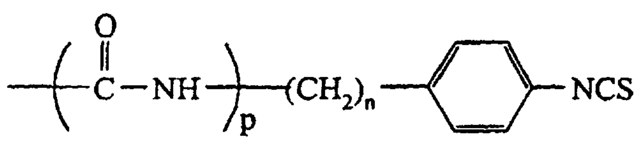 ;

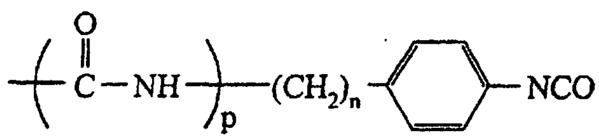 ;

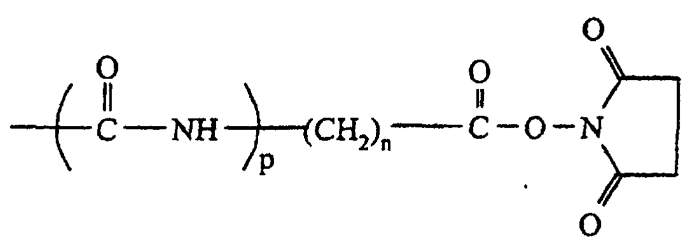 ;

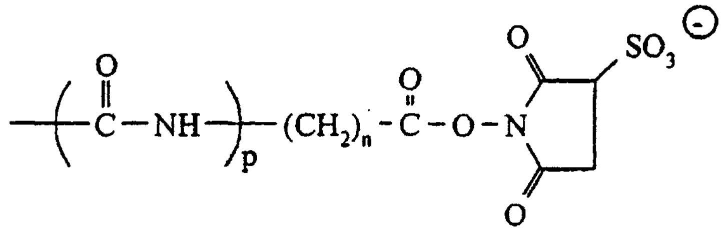 ;

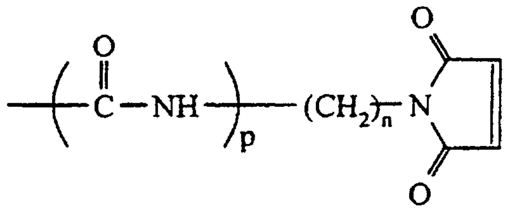 ;

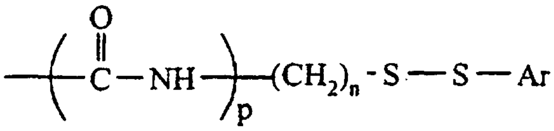 ; 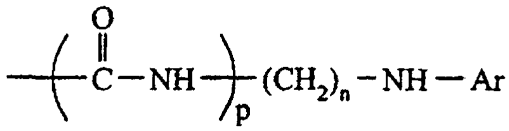 ;

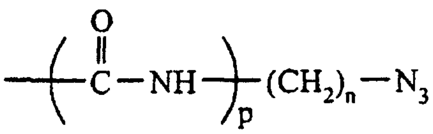 ; 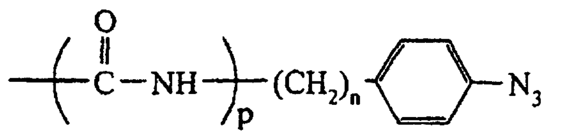 ;

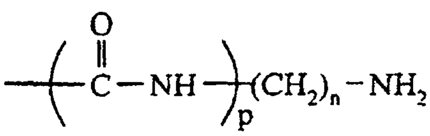 ou 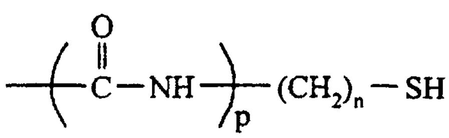

où n varie de 0 à 8 et p est égal à 0 ou 1, et Ar est un hétérocycle à 5 ou 6 chaînons comprenant 1 à 3 hétéroatomes, éventuellement substitué par un atome d'halogène, ceux des groupes $R_1$ à $R_7$ ne représentant pas une des entités réactives ci-dessus étant choisis parmi l'hydrogène ou un groupe $-(CH_2)_r-Z$ dans lequel r varie de 0 à 4 et Z représente un groupe $CH_3$, $SO_3H$, OH ou $N^+R_1R_2R_3$ dans lequel $R_1$, $R_2$ et $R_3$ sont tels que définis ci-dessus, et au moins un des groupes $R_8$ et $R_9$ représente un groupe $SO_3^-$ ou $SO_3H$, l'autre groupe représentant l'hydrogène ou un groupe $SO_3^-$ ou $SO_3H$.

**8.** Procédé selon la revendication 7, dans lequel le réactif fluorophore est une cyanine de structure :

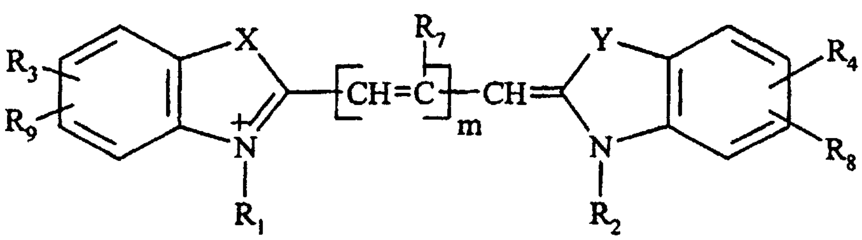

dans laquelle $R_1$, $R_2$, $R_3$, $R_4$, $R_7$, $R_8$ et $R_9$ sont tels que définis dans la revendication 7.

**9.** Procédé selon la revendication 8, dans lequel X et Y représentent chacun un groupe $C(CH_3)_2$ dans la structure de la cyanine.

**10.** Procédé selon la revendication 9, dans lequel dans la structure de la cyanine,

- $R_1$ et $R_2$ représentent chacun un groupe

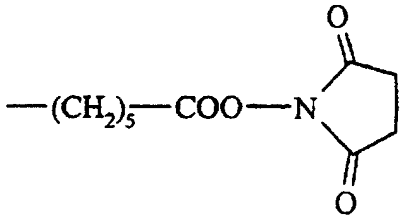 ;

- $R_3$, $R_4$ et $R_7$ représentent chacun l'hydrogène ;
- $R_8$ et $R_9$ représentent chacun un groupe $SO_3^-$ ; et
- m est égal à 2.

**11.** Procédé selon la revendication 9, dans lequel dans la structure de la cyanine,

- $R_1$ représente un groupe

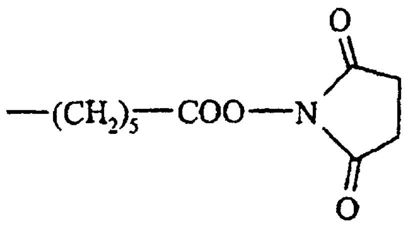 ;

- $R_2$ représente un éthyl ;
- $R_3$, $R_4$ et $R_7$ représentent chacun l'hydrogène ;
- $R_8$ et $R_9$ représentent chacun un groupe $SO_3$- ; et
- m est égal à 2.

**12.** Procédé selon l'une quelconque des revendications 1 à 11, dans lequel la molécule porteuse est un anticorps, un antigène, une protéine, un peptide, un haptène, une lectine, l'avidine, la streptavidine, une toxine, un glucide, un oligosaccharide, un polysaccharide, un acide nucléique, une hormone, un médicament, un polymère, une particule polymérique, du verre, une particule de verre ou une surface en verre ou en polymère.

**13.** Conjugué fluorescent, obtenu par le procédé de l'une quelconque des revendications 1 à 12.

**14.** Conjugué fluorescent selon la revendication 13, issu de l'accrochage du complexe d'inclusion formé par le macrocycle soluble dans l'eau et le réactif fluorophore sur la molécule porteuse.

**15.** Conjugué fluorescent selon la revendication 14, dans lequel le complexe d'inclusion subsiste dans la structure finale du conjugué et forme un rotaxane stable.

**16.** Conjugué fluorescent selon la revendication 13, issu de l'accrochage du complexe d'inclusion formé par le macrocycle soluble dans l'eau et la molécule porteuse sur le réactif fluorophore.

**17.** Conjugué fluorescent selon la revendication 16, dans lequel le complexe d'inclusion subsiste dans la structure finale du conjugué et forme un rotaxane stable.

**18.** Utilisation d'un conjugué fluorescent selon l'une quelconque des revendications 13 à 17 comme traceur fluorescent.

**19.** Utilisation selon la revendication 18, pour la détection et/ou la détermination par fluorescence d'un analyte dans un milieu susceptible de le contenir.

**20.** Utilisation selon la revendication 18, en microscopie de fluorescence ou en cytométrie de flux.

## Claims

1. A process for the obtention of a fluorescent conjugate between a carrier molecule possessing at least one amino, hydroxyl, carboxyl and/or sulfhydryl group and a fluorophoric reagent possessing at least one functional group capable of reacting with said amino, hydroxyl, carboxyl and/or sulfhydryl group(s), which consists in bringing said carrier molecule and said fluorophoric reagent into contact with an aqueous solution of a water-soluble macrocycle containing from 1 to 40% (w/v) of said macrocycle.

2. A process according to claim 1 in which the water-soluble macrocycle is selected from an optionally substituted cyclodextrin and a calixarene substituted by hydrophilic groups.

3. A process according to claim 2 in which the water-soluble macrocycle is a cyclodextrin selected from α-cyclodextrin, β-cyclodextrin, γ-cyclodextrin, hydroxypropyl-α-cyclodextrin, hydroxypropyl-β-cyclodextrin, hydroxypropyl-γ-cyclodextrin, hydroxyethyl-α-cyclodextrin, hydroxyethyl-β-cyclodextrin, hydroxyethyl-γ-cyclodextrin and 2,6-di-O-methylheptakis-β-cyclodextrin.

4. A process according to claim 2 in which the water-soluble macrocycle is a calixarene of the structure

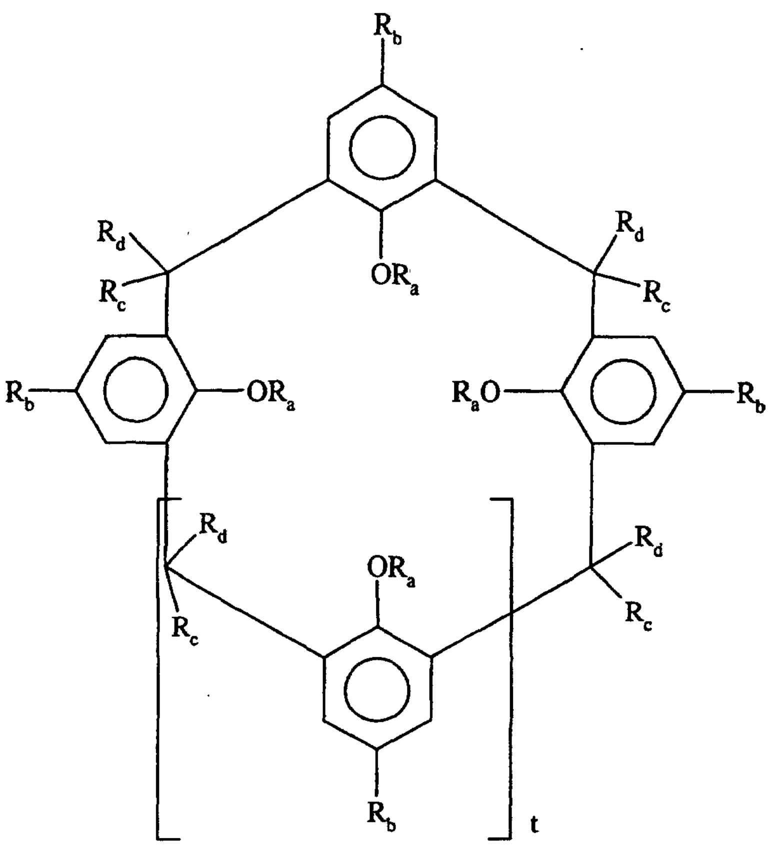

in which:

- $R_a$ and $R_b$ are each a hydrophilic group selected from H, $(CH_2)_pCO_2H$, $(CH_2)_pOH$, $(CH_2)_pNH_2$, $(CH_2)_pSO_3H$ and $(CH_2)_pN^+R_cR_dR_e$;
- $R_c$, $R_d$ and $R_e$ are each hydrogen or a $(C_1$-$C_3)$alkyl;
- p varies from 0 to 4 for $R_b$ and from 1 to 4 for $R_a$; and
- t varies from 1 to 5.

5. A process according to any one of claims 1 to 4 in which the aqueous solution of macrocycle contains from 10 to 40% (w/v) of macrocycle.

6. A process according to any one of claims 1 to 5 in which the fluorophoric reagent is a chromophore containing one or more aromatic rings and possessing a high molecular extinction coefficient.

7. A process according to claim 6 in which the fluorophoric reagent is selected from:

* a cyanin of the structure

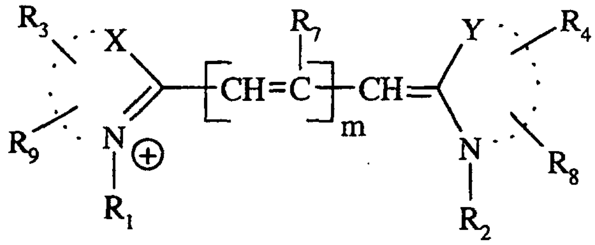

;

* a hemicyanin of the structure

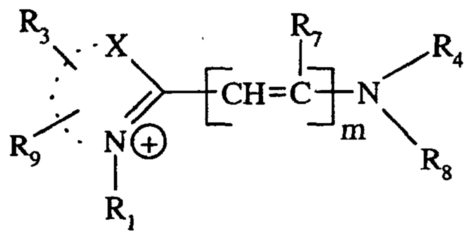

;

* a merocyanin of the structure

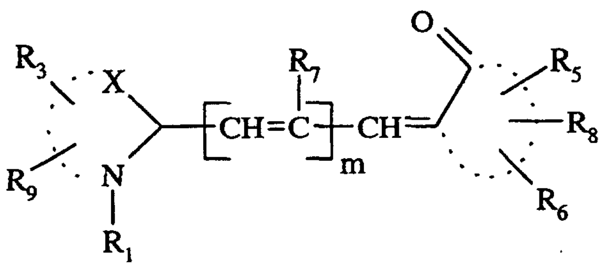

or

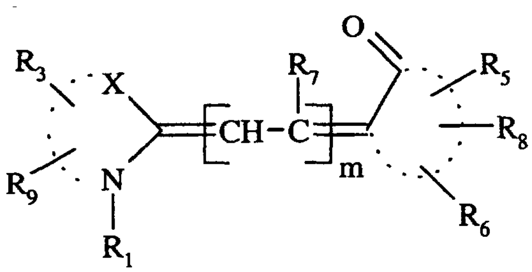

;

* a styryl compound of the structure

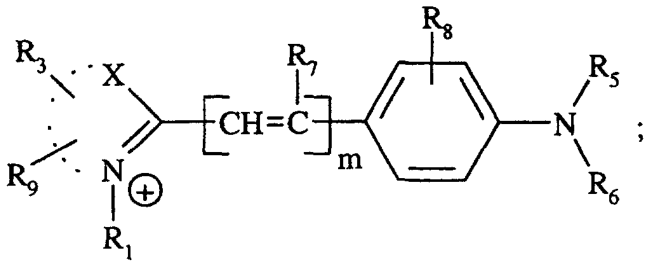

* a rhodamine of the structure

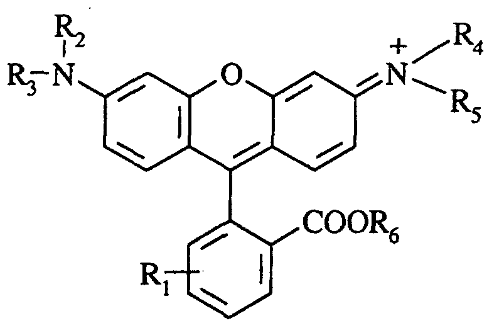

or

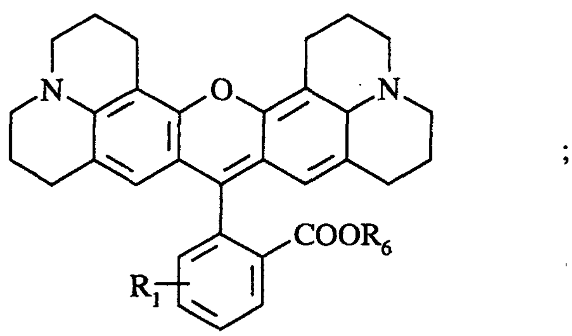

* a fluorescein of the structure

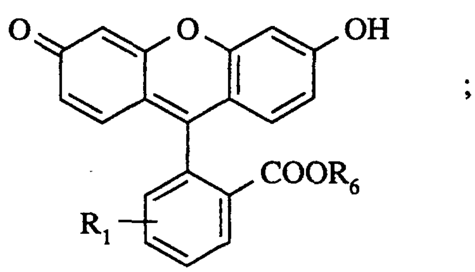

and

* a naphthofluorescein of the structure

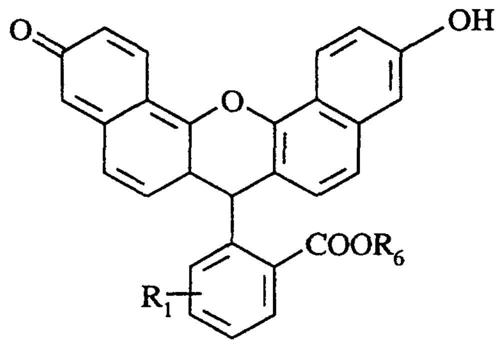 ;

in which structures:

- the broken lines each represent the carbon atoms required to form 1 to 3 fused rings, the groups $R_3$, $R_4$, $R_5$, $R_6$, $R_8$ and $R_9$ being attached to these rings;
- X and Y are each N,

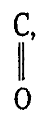

O, S or $C(CH_3)_2$;
- m has a value of 1, 2, 3 or 4;
- at least one of the groups $R_1$ to $R_7$ is capable of reacting with an amino, hydroxyl, carboxyl and/or sulfhydryl group and is selected from:

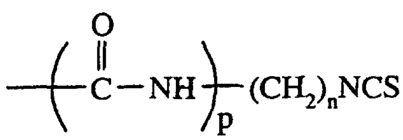 ; 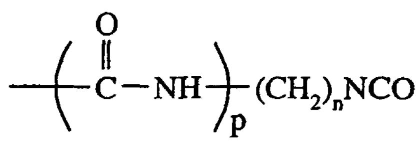 ;

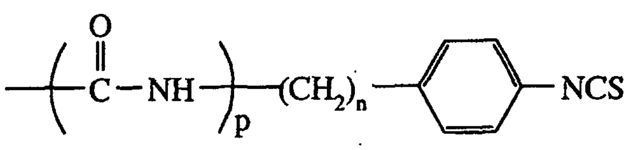 ;

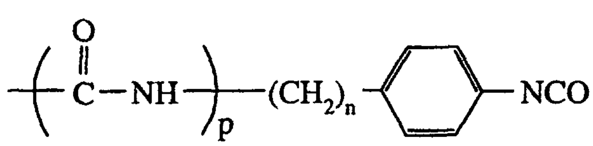 ;

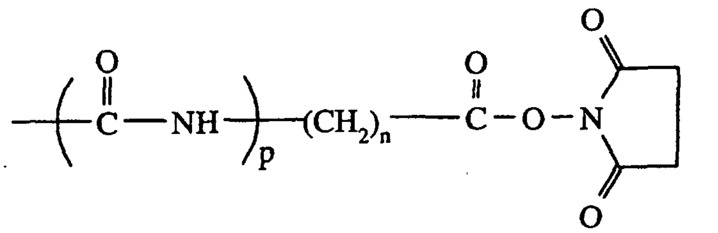 ;

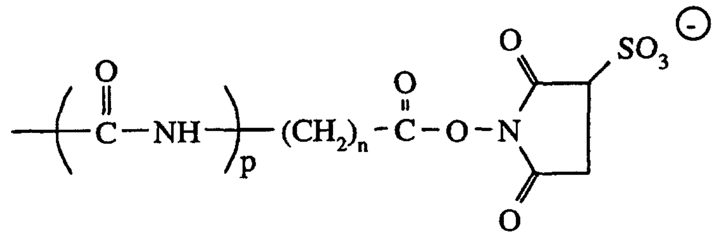 ;

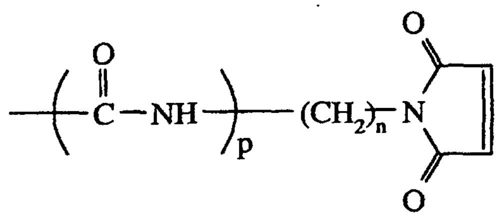 ;

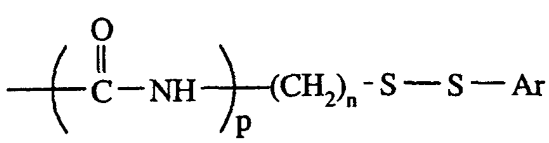 ; 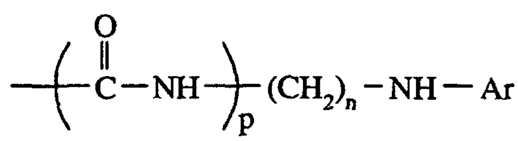 ;

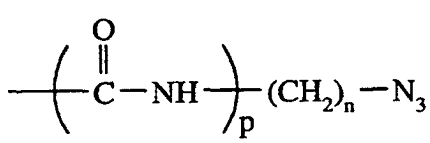 ; 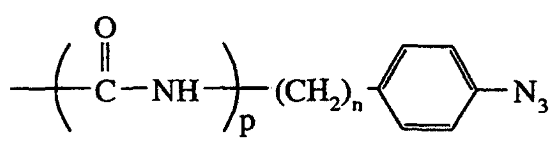 ;

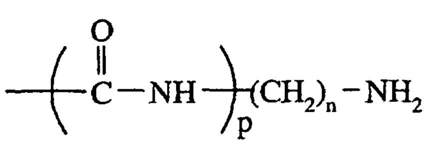 and 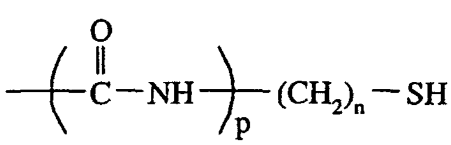

in which n varies from 0 to 8, p is equal to 0 or 1 and Ar is a 5- or 6-membered heterocycle comprising 1 to 3 heteroatoms which is optionally substituted by a halogen atom, those of the groups $R_1$ to $R_7$ which do not represent one of the above reactive entities being selected from hydrogen and a group $-(CH_2)_r-Z$, in which r varies from 0 to 4 and Z is a group $CH_3$, $SO_3H$, OH or $N^+R_1R_2R_3$, in which $R_1$, $R_2$ and $R_3$ are as defined above, and at least one of the groups $R_8$ and $R_9$ is a group $SO_3^-$ or $SO_3H$, the other group being hydrogen or a group $SO_3^-$ or $SO_3H$.

**8.** A process according to claim 7 in which the fluorophoric reagent is a cyanin of the structure

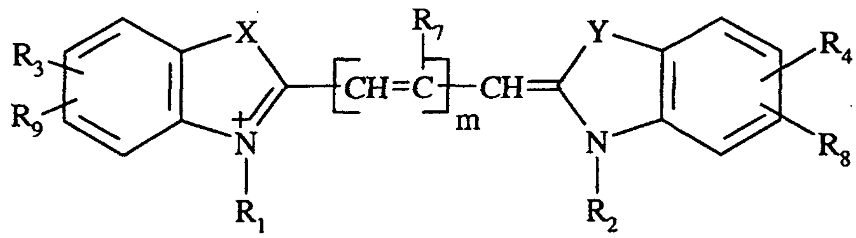

in which $R_1$, $R_2$, $R_3$, $R_4$, $R_7$, $R_8$ and $R_9$ are as defined in claim 7.

**9.** A process according to claim 8 in which X and Y are each a group $C(CH_3)_2$ in the structure of the cyanin.

**10.** A process according to claim 9 in which, in the structure of the cyanin:

- $R_1$ and $R_2$ are each a group

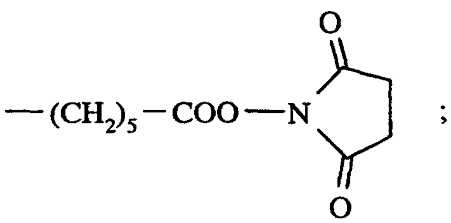

- $R_3$, $R_4$ and $R_7$ are each hydrogen;
- $R_8$ and $R_9$ are each a group $SO_3^-$; and
- m is equal to 2.

**11.** A process according to claim 9 in which, in the structure of the cyanin:

- $R_1$ is a group

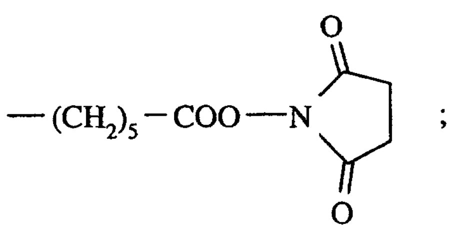

- $R_2$ is an ethyl;
- $R_3$, $R_4$ and $R_7$ are each hydrogen;
- $R_8$ and $R_9$ are each a group $SO_3^-$; and
- m is equal to 2.

**12.** A process according to any one of claims 1 to 11 in which the carrier molecule is an antibody, an antigen, a protein, a peptide, a hapten, a lectin, avidin, streptavidin, a toxin, a carbohydrate, an oligosaccharide, a polysaccharide, a nucleic acid, a hormone, a medicament, a polymer, a polymeric particle, glass, a glass particle or a glass or polymer surface.

**13.** A fluorescent conjugate obtained by the process of any one of claims 1 to 12.

**14.** A fluorescent conjugate according to claim 13 produced by attachment of the inclusion complex formed by the

water-soluble macrocycle and the fluorophoric reagent to the carrier molecule.

**15.** A fluorescent conjugate according to claim 14 in which the inclusion complex remains in the final structure of the conjugate and forms a stable rotaxane.

**16.** A fluorescent conjugate according to claim 13 produced by attachment of the inclusion complex formed by the water-soluble macrocycle and the carrier molecule to the fluorophoric reagent.

**17.** A fluorescent conjugate according to claim 16 in which the inclusion complex remains in the final structure of the conjugate and forms a stable rotaxane.

**18.** Use of a fluorescent conjugate according to any one of claims 13 to 17 as a fluorescent tracer.

**19.** Use according to claim 18 for the detection and/or determination, by fluorescence, of an analyte in a medium which may contain it.

**20.** Use according to claim 18 in fluorescence microscopy or in flow cytometry.

**Patentansprüche**

**1.** Verfahren zur Herstellung eines fluoreszierenden Konjugats zwischen einem Trägermolekül mit mindestens einer Amino-, Hydroxyl-, Carboxylund/oder Sulfhydrylgruppe und einem Fluorophor-Reagenz mit mindestens einer funktionellen Gruppe, die zur Reaktion mit der bzw. den Amino-, Hydroxyl-, Carboxyl- und/oder Sulfhydrylgruppe(n) befähigt ist, bei dem man das Trägermolekül und das Fluorophor-Reagenz mit einer wäßrigen Lösung eines wasserlöslichen Makrocyclus, die 1 bis 40% (w/v) des Makrocyclus enthält, zusammengibt.

**2.** Verfahren nach Anspruch 1, bei dem man als wasserlöslichen Makrocyclus ein gegebenenfalls substituiertes Cyclodextrin oder ein mit hydrophilen Gruppen substituiertes Calixaren einsetzt.

**3.** Verfahren nach Anspruch 2, bei dem man als wasserlöslichen Makrocyclus ein unter α-Cyclodextrin, β-Cyclodextrin, γ-Cyclodextrin, Hydroxypropyl-α-cyclodextrin, Hydroxypropyl-β-cyclodextrin, Hydroxypropyl-γ-cyclodextrin, Hydroxyethyl-α-cyclodextrin, Hydroxyethyl-β-cyclodextrin, Hydroxyethyl-γ-cyclodextrin und 2,6-Di-O-methyl-heptakis-β-cyclodextrin ausgewähltes Cyclodextrin einsetzt.

**4.** Verfahren nach Anspruch 2, bei dem man als wasserlöslichen Makrocyclus ein Calixaren der Struktur

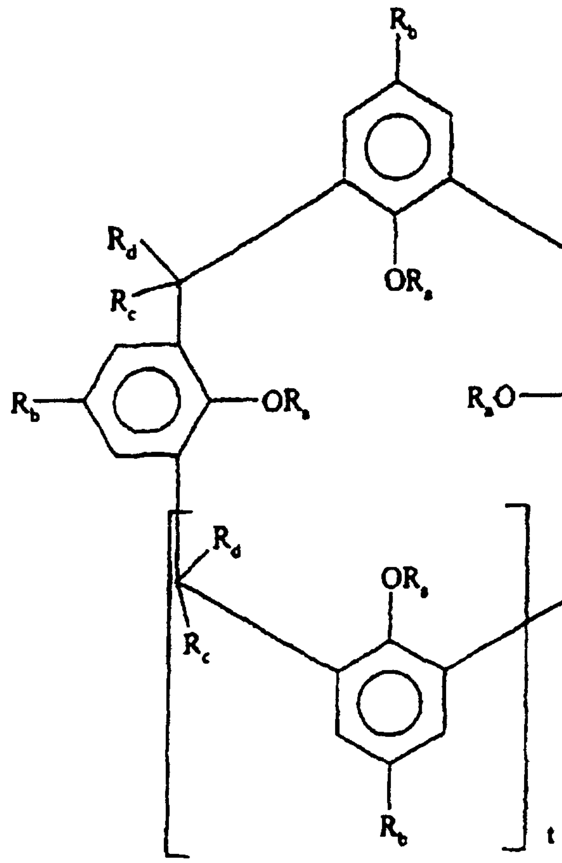

worin:

- $R_a$ und $R_b$ jeweils für eine hydrophile Gruppe stehen, die unter H, $(CH_2)_pCO_2H$, $(CH_2)_pOH$, $(CH_2)_pNH_2$, $(CH_2)_pSO_3H$ und $(CH_2)_pN^+R_cR_dR_e$ ausgewählt ist;
- $R_c$, $R_d$ und $R_e$ jeweils für Wasserstoff oder $C_1$-$C_3$-Alkyl stehen;
- p für $R_b$ im Bereich von 0 bis 4 und für $R_a$ im Bereich von 1 bis 4 liegt und
- t im Bereich von 1 bis 5 liegt;

einsetzt.

**5.** Verfahren nach einem der Ansprüche 1 bis 4, bei dem die wäßrige Lösung des Makrocyclus 10 bis 40% (w/v) Makrocyclus enthält.

**6.** Verfahren nach einem der Ansprüche 1 bis 5, bei dem man als Fluorophor-Reagenz ein Chromophor mit einem oder mehreren aromatischen Kernen mit großem molarem Extinktionskoeffizienten einsetzt.

**7.** Verfahren nach Anspruch 6, bei dem man das Fluorophor-Reagenz unter

* einem Cyanin der Struktur

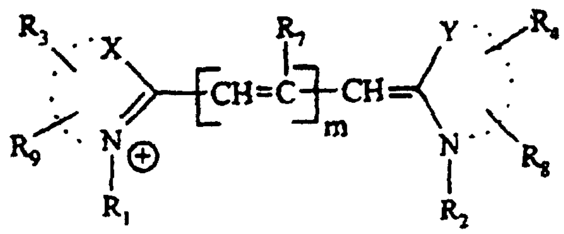

* einem Hemicyanin der Struktur

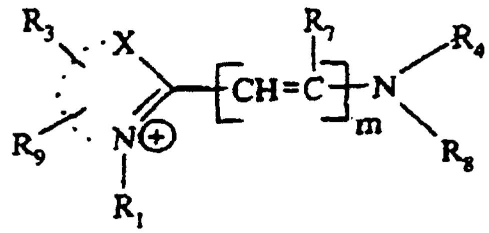

* einem Merocyanin der Struktur

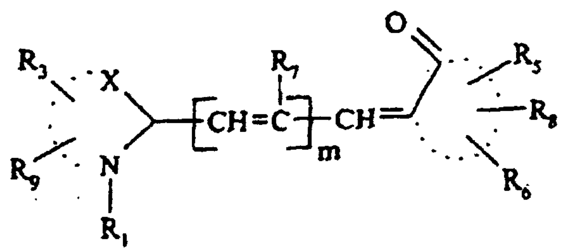

oder

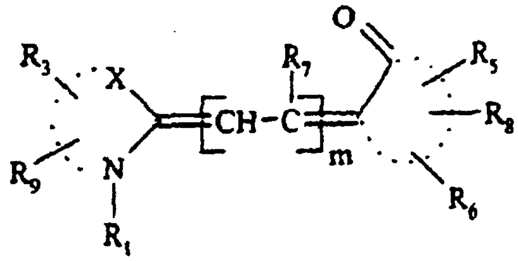

* einem Styryl der Struktur

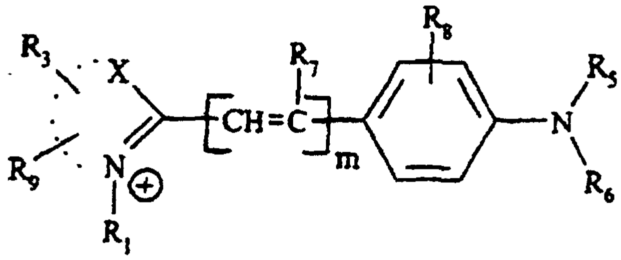

* einem Rhodamin der Struktur

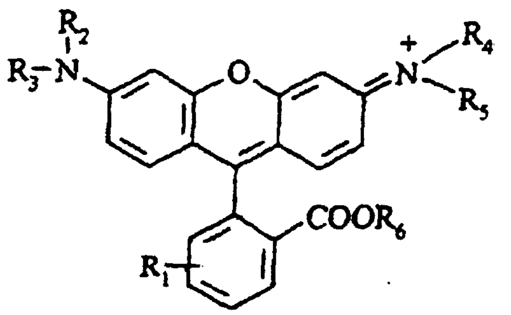

oder

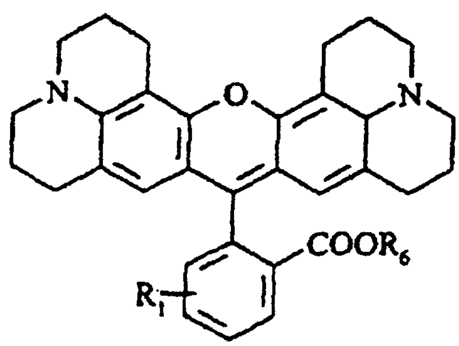

* einem Fluorescein der Struktur

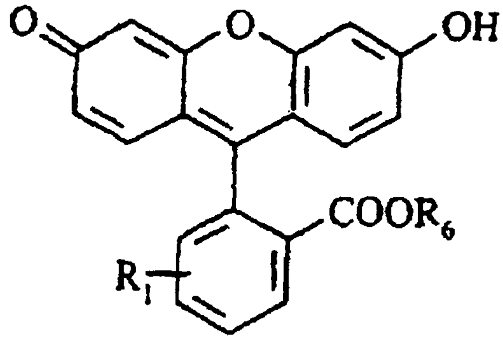

und

* einem Naphthofluorescein der Struktur

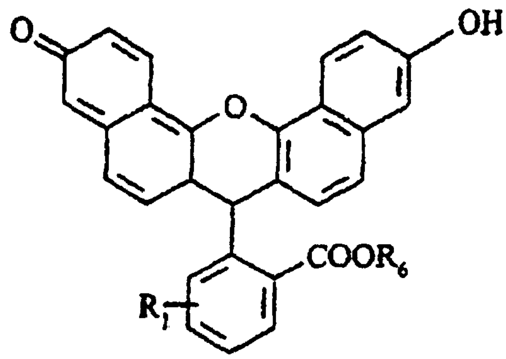

auswählt, wobei in diesen Strukturen

- die punktierten Linien jeweils die zur Bildung von 1 bis 3 anellierten Cyclen notwendigen Kohlenstoffatome darstellen, wobei die Gruppen $R_3$, $R_4$, $R_5$, $R_6$, $R_8$ und $R_9$ an diese Cyclen gebunden sind;

- X und Y jeweils für N,

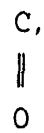

  O, S oder $C(CH_3)_2$ stehen,

- m gleich 1, 2, 3 oder 4 ist,

- mindestens eine der Gruppen $R_1$ bis $R_7$ zur Reaktion mit einer Amino-, Hydroxyl-, Carboxylund/oder Sulfhydrylgruppe befähigt ist und unter

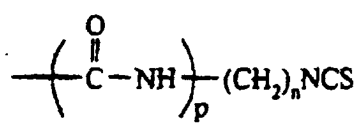 ; 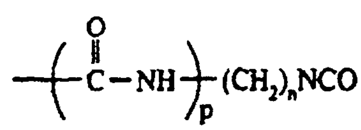 ;

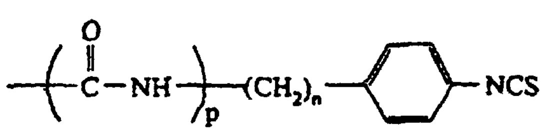 ;

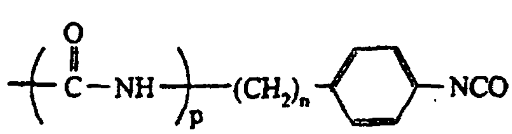 ;

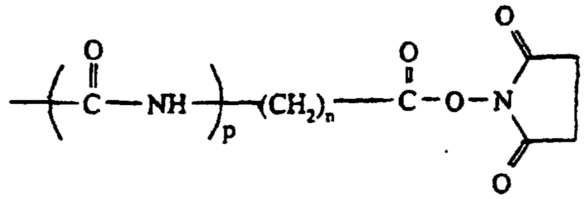 ;

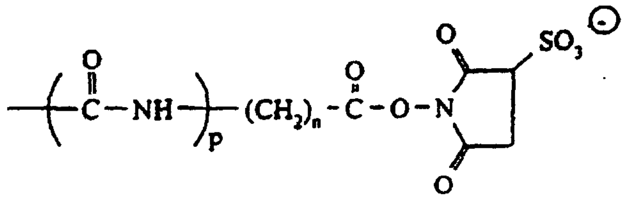 ;

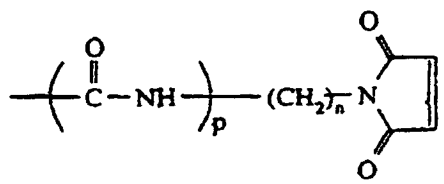 ;

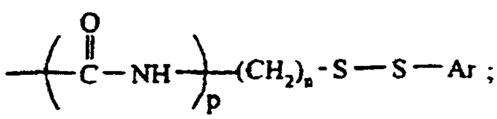 ; 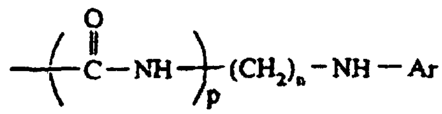 ;

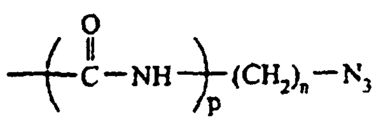 ; 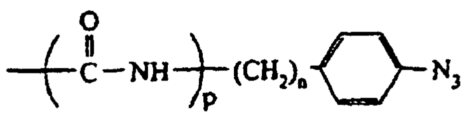 ;

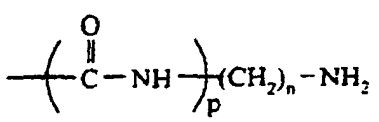 und 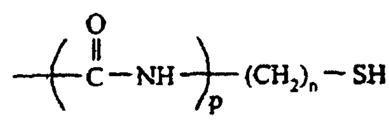

ausgewählt ist, wobei n im Bereich von 0 bis 8 liegt und p gleich 0 oder 1 ist und Ar für einen gegebenenfalls durch ein Halogenatom substituierten 5- bis 6gliedrigen Heterocyclus steht, wobei diejenigen Gruppen $R_1$ bis $R_7$, die nicht für eine der obigen reaktiven Einheiten stehen, unter Wasserstoff und einer Gruppe $-(CH_2)_r-Z$, worin r im Bereich von 0 bis 4 liegt und Z für eine $CH_3$-, $SO_3H$-, OH-Gruppe oder $N^+R_1R_2R_3$, worin $R_1$, $R_2$ und $R_3$ die oben angegebene Bedeutung besitzen, steht, ausgewählt sind und mindestens eine der Gruppen $R_8$ und $R_9$ für eine $SO_3^-$- oder $SO_3H$-Gruppe steht, wobei die andere Gruppe für Wasserstoff oder eine $SO_3^-$- oder $SO_3H$-Gruppe steht.

**8.** Verfahren nach Anspruch 7, bei dem man als Fluorophor-Reagenz ein Cyanin der Struktur

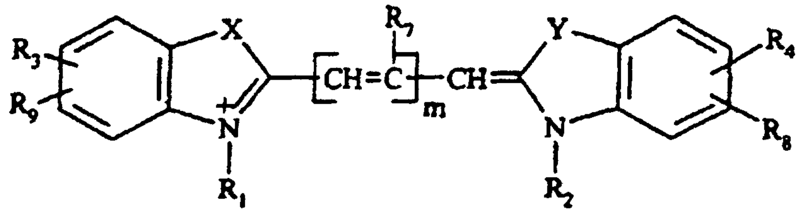

einsetzt, wobei $R_1$, $R_2$, $R_3$, $R_4$, $R_7$, $R_8$ und $R_9$ die in Anspruch 7 angegebene Bedeutung besitzen.

**9.** Verfahren nach Anspruch 8, bei dem in der Cyaninstruktur X und Y jeweils für eine $C(CH_3)_2$-Gruppe stehen.

**10.** Verfahren nach Anspruch 9, bei dem in der Cyaninstruktur

- $R_1$ und $R_2$ jeweils für eine Gruppe

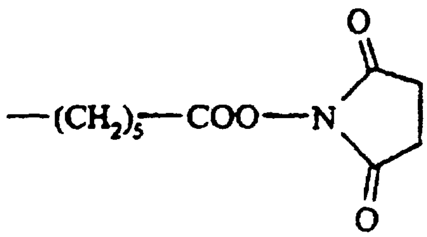

stehen;

- $R_3$, $R_4$ und $R_7$ jeweils für Wasserstoff stehen;
- $R_8$ und $R_9$ jeweils für eine $SO_3^-$-Gruppe stehen und
- m gleich 2 ist.

**11.** Verfahren nach Anspruch 9, bei dem in der Cyaninstruktur

- $R_1$ für eine Gruppe

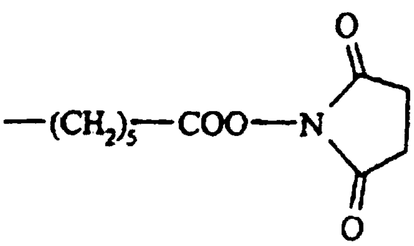

steht;

- $R_2$ für Ethyl steht;
- $R_3$, $R_4$ und $R_7$ jeweils für Wasserstoff stehen;
- $R_8$ und $R_9$ jeweils für eine $SO_3^-$-Gruppe stehen und

- m gleich 2 ist.

**12.** Verfahren nach einem der Ansprüche 1 bis 11, bei dem man als Trägermolekül einen Antikörper, ein Antigen, ein Protein, ein Peptid, ein Hapten, ein Lectin, Avidin, Streptavidin, ein Toxin, ein Kohlenhydrat, ein Oligosaccharid, ein Polysaccharid, eine Nucleinsäure, ein Hormon, einen Arzneistoff, ein Polymer, ein polymeres Teilchen, Glas, ein Glasteilchen oder eine Glasoder Polymeroberfläche einsetzt.

**13.** Fluoreszierendes Konjugat, erhalten nach dem Verfahren nach einem der Ansprüche 1 bis 12.

**14.** Fluoreszierendes Konjugat nach Anspruch 13, das durch die Ankopplung des aus dem wasserlöslichen Makrocyclus und dem Fluorophor-Reagenz gebildeten Einschlußkomplexes an das Trägermolekül entstanden ist.

**15.** Fluoreszierendes Konjugat nach Anspruch 14, bei dem der Einschlußkomplex in der Endstruktur des Konjugats bestehen bleibt und ein stabiles Rotaxan bildet.

**16.** Fluoreszierendes Konjugat nach Anspruch 13, das durch die Ankopplung des aus dem wasserlöslichen Makrocyclus und dem Trägermolekül gebildeten Einschlußkomplexes an das Fluorophor-Reagenz entstanden ist.

**17.** Fluoreszierendes Konjugat nach Anspruch 16, bei dem der Einschlußkomplex in der Endstruktur des Konjugats bestehen bleibt und ein stabiles Rotaxan bildet.

**18.** Verwendung eines fluoreszierenden Konjugats nach einem der Ansprüche 13 bis 17 als Fluoreszenztracer.

**19.** Verwendung nach Anspruch 18 zum Nachweis und/oder zur Bestimmung eines Analyten mittels Fluoreszenz in einem Medium, das wahrscheinlich den Analyten enthält.

**20.** Verwendung nach Anspruch 18 bei der Fluoreszenzmikroskopie oder Flußcytometrie.